# EUROPEAN PATENT APPLICATION

(11) **EP 1 905 367 A1**
(43) Date of publication of application: **02.04.2008**
(21) Application number: 06425666.2
(22) Date of filing: 28.09.2006
(51) Int. Cl.: A61B 17/72, A61B 17/68

(54) **Intramedullary osteosynthesis device**

(71) Applicant: Orthofix S.r.l., 37012 Bussolengo VR (IT)
(72) Inventor: Matovani, Matteo, 41011 Campogalliano (Modena) (IT); Rossi, Luigi, 37019 Peschiera del Garda (Verona) (IT)
(74) Representative: Botti, Mario

(57) **Abstract**

The present invention relates to an osteosynthesis device of a long bone (11) of a leg, comprising an intramedullary nail (12, 112) inserted in a hole (14) formed in the medullary canal of the bone (11) between a bottom and an entrance, and comprising a stiff and elongated body (13, 113), which is extended along a predetermined axis (X) between a distal end (18a, 118a) facing the bottom of the hole (14), and an opposite proximal end (18b, 118b) facing the entrance (16) of the hole (14). The device further comprises a locking element (15, 115) inserted in a transversal hole (17, 117) formed in a proximal zone of the nail (12, 112) and an axial stopping element (20) anchored to the bone at the entrance of the hole (14), and constituting an abutment for the proximal end (18b, 118b) of the nail (12, 112).

## Description

### Field of application

The present invention relates in general to the field of osteosynthesis of fractures of a long leg bone, such as for example the femur, or the tibia, in particular an adult's bone.

In a more particular aspect thereof, the present invention relates to an osteosynthesis device of a long bone of a leg, comprising an intramedullary nail adapted for being inserted in a hole formed in the medullary canal of the bone between a bottom and an entrance, and comprising a stiff and elongated body, which is extended along a predetermined axis between a distal end adapted for facing the hole bottom, and an opposite proximal end adapted for facing the hole entrance, and a locking element inserted in a transversal hole formed in the nail.

### Prior art

In the field mentioned hereinabove, the need of ensuring the juxtaposition of the fractured portions of a long bone of the leg is well known.

In particular, the fractured bone is perforated within the medullary canal along a predetermined axis for making a hole, called introduction hole, having a diameter slightly larger than the nail. Afterwards, when it is inserted in the hole, the nail is stabilised in the bone by means of locking elements.

Self-tapping screws, or pins, are used as locking elements, which are inserted into corresponding transversal holes formed in the nail body.

In particular, the use of at least two screws is envisaged at a proximal portion of the nail, that is, the portion in the proximity of the introduction point of the nail into the hole, in other words, the hole entrance, and the use of two more screws at a distal portion of the nail, that is, in the proximity of the end, which is opposite to the introduction point, towards the hole bottom.

Despite being advantageous from many standpoints, however the prior art nails exhibit some recognised disadvantages not yet overcome.

The main disadvantage lies in the fact that the known device does not ensure a complete stabilisation of the nail into the leg against the high axial loads the leg is subject to in the direction of the medullary canal, which are determined by the weight of a patient's body.

A destabilisation of the nail occurs in case of application of such loads which causes a destabilisation of the bone fractures, and jeopardizes the osteoshyntesis process.

For this reason, in order to distribute the high axial loads, it is customary to increase the number of locking screws with consequent increase of the perforations in the skin and in the bone, with the disadvantage of an increased risk of infections for a patient.

To avoid the perforations and reduce the invasiveness of the surgical treatment, it has also been proposed to use shape memory elements as locking elements, which are shaped as tabs and distributed on the nail surface. Such a nail is described in patent application WO 2005/094706 in the Applicant's name.

These shape memory elements are mobile between a rest position, taken during the nail insertion in the introduction hole wherein the shape memory elements are retracted on the nail surface, and a locking position wherein they protrude from the nail surface. The locking position is taken by the shape memory elements by the effect of the body temperature, when the nail is inserted in the introduction hole.

The use of shape memory elements, if on the one side solves the disadvantage of perforation by the locking screws, on the other side does not solve the disadvantage mentioned above of the reduced nail stabilisation.

In fact, the shape memory elements in locking position form a relatively small angle with the nail axis, because the protruding locking position is taken by the shape memory elements, when the nail already is in the introduction hole.

As a consequence, the axial load resistance offered by the shape memory elements sometimes is less than that of self-tapping locking screws.

The technical problem at the basis of the present invention therefore is to provide an osteosynthesis device of the type mentioned above, which allows the stabilisation of the intramedullary nail into the medullary canal with a reduced number of locking elements, even when excessive axial loads are applied on the nail.

### Summary of the invention

The solution idea at the basis of the present invention lies in strengthening the nail stabilisation by the use of an axial stop for the nail itself.

Based on this solution idea, the technical problem is solved according to the invention by a osteosynthesis device of a leg bone, of the type mentioned above, which comprises an axial stopping element adapted for being constrained to the bone at the entrance of the hole, and constituting an abutment for the proximal end of the nail.

The main advantage of the present invention lies in the fact of having a direct stop of the nail into the bone at the proximal end.

Moreover, since the axial stopping element is constrained to the bone at the hole entrance, it determines a distribution of the axial load in the area comprised between the transversal locking element and the axial stopping element itself.

An improved stability and the possibility of reducing the number of locking elements to be used, such as self-tapping screws or shape memory elements, are therefore obtained.

In a preferred solution, the axial stopping element is a body having a substantially cup shape, wherein there are defined a mantle, a bottom, and a free edge, and which form an inner cavity. The proximal end of the nail is housed in the latter.

The free -edge of the cup element carries front cutting edges for favouring the cup body advance into the bone.

Even more preferably, the cup body has a very small height, and the inner cavity occupies a prevailing portion thereof, so that the cup body, when is introduced, has relatively small axial overall dimensions that slightly exceed the overall dimensions of the nail at the proximal end thereof.

Thanks to these features, the stopping element is also light, that is, it has a very low weight as compared to the nail so as to little affect the overall weight of the device.

According to a preferred embodiment, the mantle of the cup body comprises a cylindrical portion occupying a prevailing portion thereof starting from the bottom, and having self-tapping outer threads for obtaining a threaded connection with the bone, and a self-penetrating conical portion, occupying the remaining portion towards the free edge, and capable of progressively enlarging the hole during the introduction into the hole, and preparing the female threads of the hole.

The screwing into the hole exhibits the advantage of ensuring a steady fixing of the axial stopping element into the bone, and of checking the insertion of the stopping element into the hole entrance till abutment against the proximal end of the nail.

Further features and advantages of the osteosynthesis device according to the invention will appear even more clearly from the following description of a preferred embodiment thereof, made by way of an indicative non-limiting example with reference to the annexed drawings

### Brief description of the figures

Figure 1 shows an osteosynthesis device according to the prior art implanted in a femur.
Figure 2 shows an osteosynthesis device according to the present invention implanted in a femur.
Figure 3 shows a view of another osteosynthesis device according to the present invention implanted in a femur.
Figure 4 shows an enlarged scale view of an axial stopping element of the osteosynthesis device of figures 2 and 3;
Figure 5 shows an enlarged scale section view of a detail of the device of figure 2 in a first operating condition;
Figure 6 shows an enlarged scale section view of a detail of the device of figure 2 in a second operating condition

### DETAILED DESCRIPTION

With reference to the annexed figures, an osteosynthesis device according to the invention is generally indicated with the reference number 10.

The osteosynthesis device 10 comprises an intramedullary nail 12 inserted in a hole 14, formed in the medullary canal of a long bone 11 between a bottom and an entrance 16, in particular of a femur.

The intramedullary nail 12 is made of titanium and comprises an elongated stiff body 13, or stem, extended along a predetermined axis X between a distal end 18a, i.e., facing the bottom of the hole 14 and an opposite proximal end 18b, i.e., facing the entrance 16 of the hole 14. The hole 14 has a substantially cylindrical shape of a size depending on the dimensions of the intramedullary nail 12, which has a diameter preferably comprised between 7 and 13 mm.

The intramedullary nail 12 further comprises locking screws 15 intended for locking the nail 12, and in particular, as it will be better detailed hereinafter, a locking screw 15 in the proximal zone and a locking screw 15 in the distal zone.

Each screw 15 is inserted in a respective transversal hole 17 formed in the body 13.

According to the invention, the osteosynthesis device comprises an axial stopping element 20 fixed at the entrance 16 of the hole 14 of the medullary canal and constituting an abutment for the proximal end 18b of the nail 12.

The axial stopping element 20 is an element structurally independent of the nail and is constrained to the bone only, not to the nail, so as to act as a stopping element, or arrest, for the nail 12.

In particular, the axial stopping element 20 is a cup body of titanium, wherein there are defined a cylindrical mantle 22, a bottom 21, and a free edge 25, and which form an inner cavity 35. The proximal end 18b of the nail 12 is housed in the latter.

Even more in particular, the cup body has a very small height L, substantially equal to or slightly larger than, the outer diameter D, in the example L is equal to about 21 mm, and D is equal to about 18 mm.

The inner cavity 35 occupies a prevailing portion of the cup body, and in the example has a height L1 of about 12 mm.

In the illustrated solution, the axial stopping element 20 is screwed in the hole of the medullary canal. In particular, the above mantle 22 of the cup body comprises a cylindrical portion 23, occupying a prevailing portion thereof starting from the bottom 21 and carrying self-tapping outer threads 24, and a conical portion 30, self-penetrating into the hole 14, occupying the remaining tip portion, towards the free edge 25.

Even more in particular, the free edge 25 carries front cutting edges 32 for further favouring the insertion of the axial stopping element 20, and the forming of the female threads into hole 14.

Preferably, the threads 24 has the outer diameter D of about 18 mm mentioned above, slightly larger than the diameter of the hole 14 for threading the hole 14 during the insertion.

To this end it should be noted that hole 14 is formed in a spongy portion of the bone. For this reason, the outer threads 24 of the axial stopping element 20 has thread, or ridge, pitch and depth, which are suitable- for threading the spongy portion of-the bone. In particular, the thread has a triangular shape, the pitch is 2 mm, and the depth is 1,5 mm.

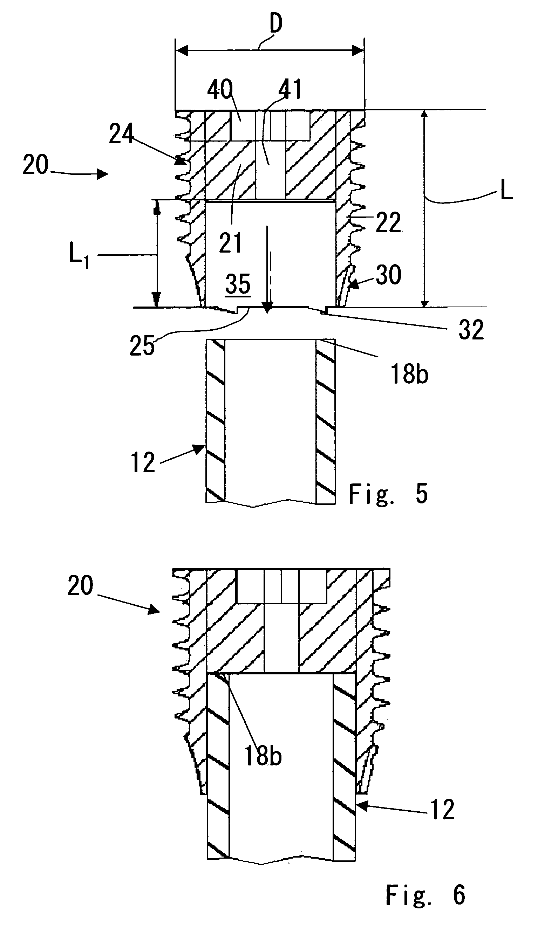

With reference to figure 5, it is further noted that the cup body is completed by a hexagonal recess 40 for being screwed into the bone by means of a special wrench, and a through hole 41 for housing a guide wire.

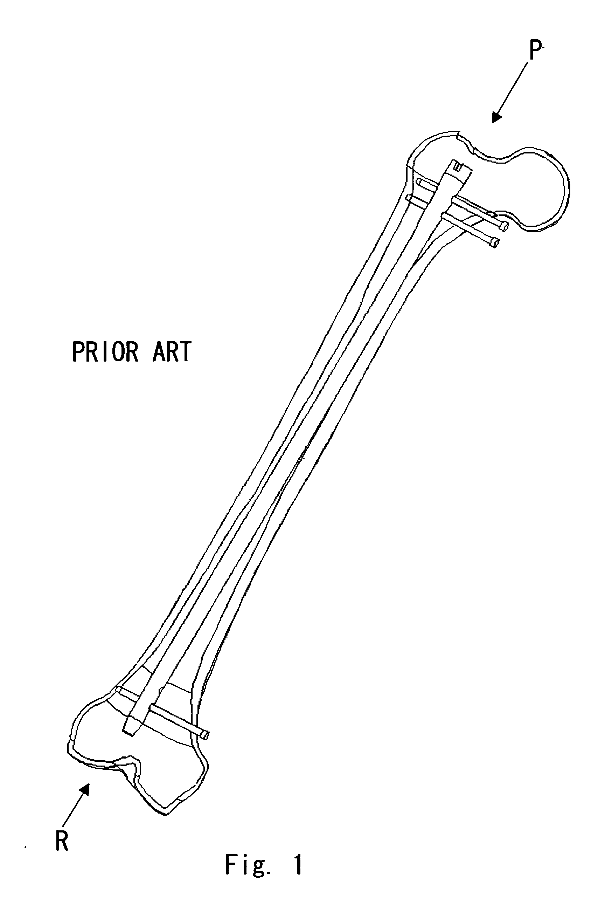

With reference to figures 1, 2 and 3, the operation of device 10 when nail 12 is inserted in the medullary canal shall now be described, and this operation is compared with that of a known device.

In particular, reference letters P and R respectively indicate the directions of the axial loads the femur is subject to, that is, the weight force of a patient, which mainly acts on the head of femur 11, and the reaction force exerted by a femur support surface and acting mainly along the medullary canal of the bone, in the direction opposite to the weight.

During the operation of the known device of figure 1, the nail 12 subject to both forces P and R, tends to move into the hole 14 towards the entrance 16, and is therefore destabilised. In this condition, the use of at least two locking screws 15 in the proximal zone is necessarily required.

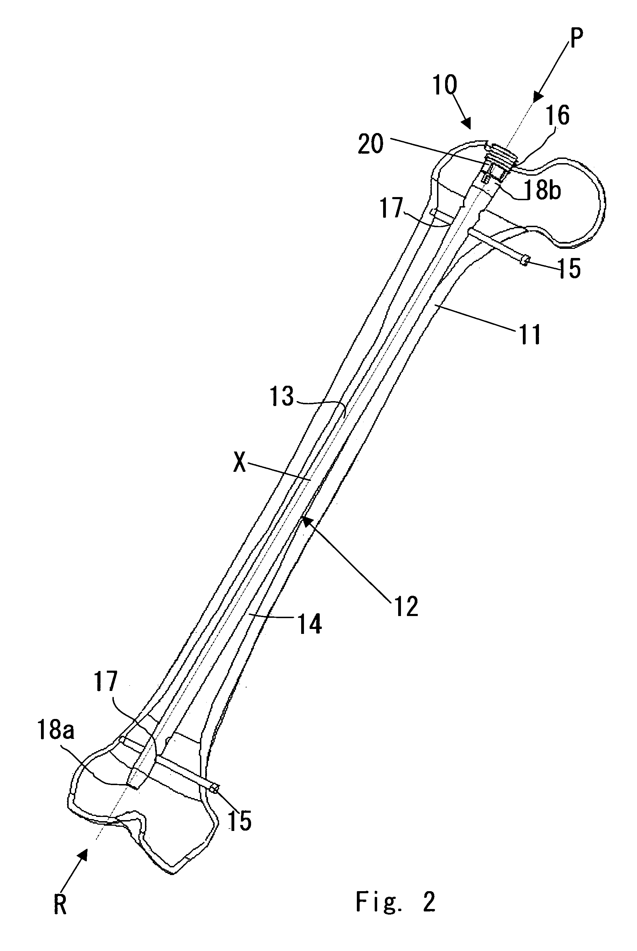

In the device 10 of figure 2 according to the invention, the nail 12 subject to the same both forces P, R is kept in correct position by the stop, or abutment, exerted by the axial stopping element 20, which as said above is fixedly constrained to the bone at the proximal end 16.

In the practice, the main advantage of the osteosynthesis device according to the present invention lies in a direct locking at the proximal end of the nail into the bone, thereby with the possibility of reducing the locking elements, in particular those that are the closest to the proximal end.

In other words, the main advantage of the device according to the invention lies in the fact-that the nail stabilisation is obtained exactly in the proximal zone. In fact, the axial stopping element is close to the proximal end, and therefore the axial load is distributed mainly between the proximal screw and the axial stopping element.

It is therefore possible to reduce the number of locking screws 15 in the proximal zone from two to one as illustrated in figure 2, so as to reduce the proximal overall dimensions of the screws in this zone, as well as the number of perforations required.
In other words, it is possible to use a single locking screw in the proximal zone.

A further advantage of the present invention lies in moderate overall dimensions and low weight of the axial stopping element, thanks to the small height of the axial stopping element and to the fact that the inner cavity occupies a prevailing portion thereof.

The height of the axial stopping element and the presence of the inner cavity further allow checking the screwing within axis into the introduction hole and obtaining a guided insertion of the proximal end of the nail.

The inner cavity has also the advantage of ensuring a steady reciprocal positioning of the axial stopping element and of the nail, when the device is inserted in the bone avoiding side parasitic movements of the proximal portion of the nail.

The axial stopping element also exhibits the advantage of protecting the nail from the fibrous absorption caused by the periosteum at the proximal end, which makes the removal of the nail itself difficult.

The reduction of the overall dimensions of the locking screws has also the advantage of increasing the number and type of fractures that can be treated with the device according to the invention, allowing the treatment of high femur and subtronchanteric fractures.

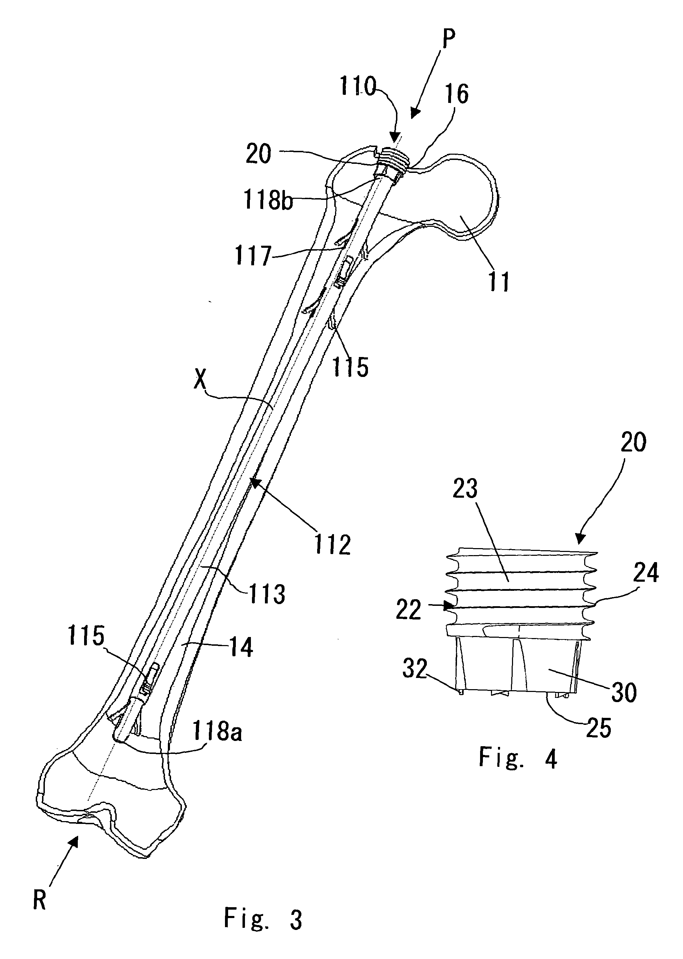

All of these advantages are even more evident with reference to figure 3 which shows an osteosynthesis device 110 according to the invention comprising a so-called shape memory intramedullary nail 112, that is, provided with shape memory locking elements 115 distributed on the surface of the nail 112.

The nail 112, which like the previous one comprises an elongated body 113 extended along an axis between a distal tip end 118a and a proximal end 118b, and wherein each shape memory locking element 115 is inserted in a corresponding transversal hole 117 formed in the body of nail 112, is widely described in the aforementioned international patent application WO 2005/094706 in the Applicant's name.

The nail 112 is described in particular in the embodiment illustrated in figures 1-15d of the above international patent application.

The osteosynthesis device according to the invention comprises an axial stopping element 20, having the same cup body shape and the same features as the axial stopping element 20 described above, and arranged as an abutment against the proximal end 118b of the nail 112.

Also in the solution of figure 3, the proximal end 118b of the nail 112 is inserted in a guided manner in the inner cavity 35 of the axial stopping element 20.

Thanks to the axial stopping element 20, the nail 112 is stopped from possible movements which could occur and which would not be prevented by the shape memory elements 115, due to the low resistance, in the axial direction towards the entrance 16.

In the solution of figure 3, the improved stabilisation in the proximal zone allows a reduction of the number of shape memory elements 115 of the nail 112 used in this zone, to the advantage of a reduction of the nail manufacturing cost.

It is clear that a man skilled-in the art may make several changes and variations to the device described -above in order to meet specific and incidental needs, all falling within the scope of protection of the invention as defined by the following claims.

## Claims

1. Osteosynthesis device -of a long bone (11) of a leg, comprising an intramedullary nail (12, 112) adapted for being inserted in a hole (14) formed in the medullary canal of the bone (11) between a bottom and an entrance (16), and comprising a stiff and elongated body (13, 113), which is extended along a predetermined axis (X) between a distal end (18a, 118a) adapted for facing the bottom of the hole (14), and an opposite proximal end (18b, 118b) adapted for facing the entrance (16) of the hole (14), and a locking element (15, 115) inserted in a transversal hole (17, 117) formed in the nail (12, 112), **characterised in that** it comprises an axial stopping element (20) adapted for being constrained to the bone at the entrance (16) of the hole (14) and constituting an abutment for the proximal end (18b, 118b) of the nail (12, 112).

2. Osteosynthesis device according to claim 1, **characterised in that** the axial stopping element (20) is a body having a substantially cup shape, which is defined by a mantle (22), a bottom (21) forming a stopping abutment for the nail (12), and a free edge (25), which form an inner cavity (35).

3. Osteosynthesis device according to claim 2, **characterised in that** the mantle (22) of the cup body carries an outer threads (24) suitable for being screwed into the bone.

4. Osteosynthesis device according to claim 3, **characterised in** -that the mantle (22) of the cup body comprises a cylindrical portion (23), occupying a prevailing portion of the cup body starting from the bottom (21) and carrying said outer threads (24) and a conical portion (30) with self-penetrating function into the bone, occupying a remaining portion towards the free edge (25) of the cup body.

5. Osteosynthesis device according to claim 4, **characterised in that** the cup body has predetermined height (L) and outer diameter (D), wherein the height (L) has a value substantially equal to or slightly larger than the outer diameter (D).

6. Osteosynthesis device according to claim 2, **characterised in that** the inner cavity (35) occupies a prevailing portion of the cup body.

7. Osteosynthesis device according to claim 1, **characterised in that** the axial stopping element (20) comprises a through hole (41) for housing a guide wire.

8. Osteosynthesis device according to claim 1, **characterised in that** it comprises a single locking element (15) in a portion of the nail facing the proximal end (18b), and said locking element is a screw.

9. Osteosynthesis device according to claim 1, **characterised in that** said locking element is a shape memory element (115).
